# EUROPEAN PATENT SPECIFICATION

(11) **EP 1 641 483 B1**
(45) Date of publication and mention of the grant of the patent: **13.02.2008**
(21) Application number: 04753440.9
(22) Date of filing: 10.06.2004
(51) Int. Cl.: A61K 38/17, C12N 15/62, C07K 14/47

(54) **FUSION PROTEINS**
FUSIONSPROTEINE
PROTEINES DE FUSION

(30) Priority: 12.06.2003 US 477880 P; 13.05.2004 US 570908 P
(43) Date of publication of application: 05.04.2006
(73) Proprietor: ELI LILLY AND COMPANY, Indianapolis, Indiana 46285 (US)
(72) Inventor: GLAESNER, Wolfgang, Carmel, IN 46032 (US); MILLICAN, Rohn, Lee, Jr., Indianapolis, IN 46259 (US); TIAN, Yu, Carmel, IN 46033 (US); TSCHANG, Sheng-Hung, Rainbow, Carmel, IN 46033 (US); VICK, Andrew, Mark, Fishers, IN 46037 (US)
(74) Representative: Kent, Lindsey Ruth
(86) International application number: PCT/US2004/016611
(87) International publication number: WO 2004/110472

(56) References cited:
- WO-A-02/46227
- WO-A-96/04388
- WO-A-97/00319
- WO-A-97/28267
- ANGAL S ET AL: "A SINGLE AMINO ACID SUBSTITUTION ABOLISHES THE HETEROGENEITY OF CHIMERIC MOUSE/HUMAN (IGG4) ANTIBODY" MOLECULAR IMMUNOLOGY, ELMSFORD, NY, US, vol. 30, no. 1, January 1993 (1993-01), pages 105-108, XP000770218 ISSN: 0161-5890
- REDDY ET AL: "Elimination of Fc Receptor-Dependent Effector Functions of a Modified IgG4 Monoclonal Antibody to Human CD4" J. IMMUNOL., vol. 164, 2000, pages 1925-1933,

### SEQUENCE LISTING

<110> Eli Lilly and Company
<120> Fusion proteins
<130> X-16821
<150> 60/477880
   <151> 2003-06-12
<150> 60/570908
   <151> 2004-05-13
<160> 8
<170> PatentIn version 3.3
<210> 1
   <211> 230
   <212> PRT
   <213> Artificial
<220>
   <223> synthetic Construct
<220>
   <221> MISC_FEATURE
   <222> (1)..(1)
   <223> xaa at position 1 is Ala or absent
<220>
   <221> MISC_FEATURE
   <222> (16)..(16)
   <223> xaa at position 16 is Pro or Glu
<220>
   <221> MISC_FEATURE
   <222> (17)..(17)
   <223> Xaa at position 17 is Phe, Val, or Ala
<220>
   <221> MISC_FEATURE
   <222> (18)..(18)
   <223> Xaa at position 18 is Leu, Glu, or Ala
<220>
   <221> MISC_FEATURE
   <222> (80)..(80)
   <223> xaa at position 80 is Asn or Ala
<220>
   <221> MISC_FEATURE
   <222> (230)..(230)
   <223> Xaa at position 230 is Lys or is absent
<400> 1
   Xaa Glu Ser Lys Tyr Gly Pro Pro Cys Pro Pro Cys Pro Ala Pro Xaa
   1               5                   10                  15
   Xaa Xaa Gly Gly Pro Ser Val Phe Leu Phe Pro Pro Lys Pro Lys Asp
   20 25 30
   Thr Leu Met Ile Ser Arg Thr Pro Glu Val Thr Cys Val Val Val Asp
   35 40 45
   Val Ser Gln Glu Asp Pro Glu Val Gln Phe Asn Trp Tyr Val Asp Gly
   50 55 60
   Val Glu Val His Asn Ala Lys Thr Lys Pro Arg Glu Glu Gln Phe Xaa
   65 70 75 80
   Ser Thr Tyr Arg Val Val Ser Val Leu Thr Val Leu His Gln Asp Trp
   85 90 95
   Leu Asn Gly Lys Glu Tyr Lys Cys Lys Val Ser Asn Lys Gly Leu Pro
   100 105 110
   Ser Ser Ile Glu Lys Thr Ile Ser Lys Ala Lys Gly Gln Pro Arg Glu
   115 120 125
   Pro Gln Val Tyr Thr Leu Pro Pro Ser Gln Glu Glu Met Thr Lys Asn
   130 135 140
   Gln Val Ser Leu Thr Cys Leu Val Lys Gly Phe Tyr Pro Ser Asp Ile
   145 150 155 160
   Ala Val Glu Trp Glu Ser Asn Gly Gln Pro Glu Asn Asn Tyr Lys Thr
   165 170 175
   Thr Pro Pro Val Leu Asp Ser Asp Gly Ser Phe Phe Leu Tyr Ser Arg
   180 185 190
   Leu Thr Val Asp Lys Ser Arg Trp Gln Glu Gly Asn Val Phe Ser Cys
   195 200 205
   Ser Val Met His Glu Ala Leu His Asn His Tyr Thr Gln Lys Ser Leu
   210 215 220
   Ser Leu Ser Leu Gly Xaa
   225 230
<210> 2
   <211> 15
   <212> PRT
   <213> Artificial
<220>
   <223> synthetic Construct
<400> 2
   Gly Gly Gly Gly Ser Gly Gly Gly Gly Ser Gly Gly Gly Gly Ser
   1 5 10 15
<210> 3
   <211> 6
   <212> PRT
   <213> Homo sapiens
<400> 3
   Pro Pro Cys Pro Ser Cys
   1 5
<210> 4
   <211> 22
   <212> PRT
   <213> Artificial
<220>
   <223> synthetic Construct
<400> 4
   Gly Ser Gly Gly Gly Gly Ser Gly Gly Gly Gly Ser Gly Gly Gly Gly
   1 5 10 15
   Ser Gly Gly Gly Gly Ser
   20
<210> 5
   <211> 825
   <212> DNA
   <213> Homo sapiens
<400> 5
   cacggcgagg gcaccttcac ctccgacgtg tcctcctatc tcgaggagca ggccgccaag      60
   gaattcatcg cctggctggt gaagggcggc ggcggtggtg gtggctccgg aggcggcggc     120
   tctggtggcg gtggcagcgc tgagtccaaa tatggtcccc catgcccacc ctgcccagca     180
   cctgaggccg ccgggggacc atcagtcttc ctgttccccc caaaacccaa ggacactctc     240
   atgatctccc ggacccctga ggtcacgtgc gtggtggtgg acgtgagcca ggaagacccc     300
   gaggtccagt tcaactggta cgtggatggc gtggaggtgc ataatgccaa gacaaagccg     360
   cgggaggagc agttcaacag cacgtaccgt gtggtcagcg tcctcaccgt cctgcaccag     420
   gactggctga acggcaagga gtacaagtgc aaggtctcca acaaaggcct cccgtcctcc     480
   atcgagaaaa ccatctccaa agccaaaggg cagccccgag agccacaggt gtacaccctg     540
   cccccatccc aggaggagat gaccaagaac caggtcagcc tgacctgcct ggtcaaaggc     600
   ttctacccca gcgacatcgc cgtggagtgg gaaagcaatg ggcagccgga gaacaactac     660
   aagaccacgc ctcccgtgct ggactccgac ggctccttct tcctctacag caggctaacc     720
   gtggacaaga gcaggtggca ggaggggaat gtcttctcat gctccgtgat gcatgaggct     780
   ctgcacaacc actacacaca gaagagcctc tccctgtctc tgggt                     825
<210> 6
   <211> 30
   <212> PRT
   <213> Artificial
<220>
   <223> synthetic construct
<400> 6
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser
               20                  25                  30
<210> 7
   <211> 25
   <212> PRT
   <213> Artificial
<220>
   <223> synthetic Construct
<400> 7
   Asp Ala Ala Ala Lys Glu Ala Ala Ala Lys Asp Ala Ala Ala Arg Glu
   1               5                   10                  15
   Ala Ala Ala Arg Asp Ala Ala Ala Lys
               20                  25
<210> 8
   <211> 14
   <212> PRT
   <213> Artificial
<220>
   <223> synthetic construct
<400> 8
   Asn Val Asp His Lys Pro Ser Asn Thr Lys Val Asp Lys Arg
   1               5                   10

## Description

### FIELD OF THE INVENTION

The present invention relates to heterologous fusion proteins comprising an active therapeutic peptide and a constant heavy chain (Fc) portion of an immunoglobulin that has the effect of extending the *in vivo* half-life of the active therapeutic peptide. These heterologous fusion proteins can be used to treat human diseases as well as a variety of other conditions or disorders.

Many active therapeutic peptides show promise in clinical trials for the treatment of various diseases. However, the usefulness of therapy involving these peptides has been limited by the fact that many peptides are poorly active, rapidly cleared *in vivo,* or have extremely short *in vivo* half-lives. Various approaches have been undertaken to extend the elimination half-life of these peptides or reduce clearance of these peptides from the body while maintaining biological activity. One approach involves fusing an active therapeutic peptide to the constant heavy chain (Fc) portion of an immunoglobulin. Immunoglobulins typically have long circulating half-lives *in vivo.* For example, IgG molecules can have a half-life in humans of up to 23 days. The Fc portion of the immunoglobulin is responsible, in part, for this *in vivo* stability. These heterologous fusion proteins take advantage of the stability provided by the Fc portion of an immunoglobulin while preserving the biological activity of the peptides.

WO 97/00319 discloses chimeric proteins which comprise leptin or a mutant or variant thereof fused to a human immunoglobulin domain. WO 96/04388 discloses soluble proteins having human interleukin (IL4) and/or human interleukin 13 (IL13) antagonist or partial antagonist activity which comprise an IL4 mutant or variant fused to at least one human immunoglobulin constant domain or fragment thereof. WO 97/28267 discloses fusion proteins which comprise a peptide having CTLA4 activity fused to an immunoglobulin constant region.

Although this approach is feasible for peptide therapeutics (See, for example, WO 02/46227 which discloses heterologous fusion proteins comprising a GLP-1 compound and the Fc portion of an immunoglobulin or analog or fragment thereof), there is a general concern regarding half antibody formation, unwanted effector function, glycosylation sites, and heterogeneity expression.

Angal, et al., Mol. Immun. (1993) 30(1): 105-108 describes how human immunoglobulin G4 (IgG4) exists in two molecular forms: the H₂L₂ tetramer and the HL "half antibody", due to the heterogeneity of the inter-heavy chain disulphide bridges. It was found that a single residue substitution at position 241 (Kabat numbering system) led to the production of a homogeneous antibody. Reddy, et al., J. Immun. (2000) 164: 1925-1933 discusses the CD4 monoclonal antibody clenoliximab, which comprises a modified IgG4 containing two single residue substitutions designed to stabilize the inter-heavy chain disulphide bridges and to ablate residual Fc receptor binding.

The present invention seeks to overcome the problems described above by identifying and substituting amino acids at various positions in the Fc portion of the molecule that reduce half antibodies and lessen or eliminate effector function. In addition, the present invention also provides identifying and substituting amino acids at various positions in the Fc portion of the molecule that do not have glycosylation sites and have reduced heterogeneity during expression. Furthermore, it is desired that identifying and substituting amino acids at various positions in the Fc portion of the molecule does not induce an immune response after repeated and prolonged administration of the heterologous fusion protein.

Compounds of the present invention include heterologous fusion proteins comprising an active therapeutic peptide fused to the Fc portion of an immunoglobulin, the Fc portion comprising the sequence of SEQ ID NO: 1 wherein:
Xaa at position 1 is Ala or absent;
Xaa at position 16 is Pro or Glu;
Xaa at position 17 is Val, or Ala;
Xaa at position 18 is Ala;
Xaa at position 80 is Asn or Ala; and
Xaa at position 230 is Lys or is absent.

The peptide portion and the Fc portion of the present invention are fused directly together or via a linker. An example of a linker is a G-rich peptide linker having the sequence Gly-Gly-Gly-Gly-Ser-Gly-Gly-Gly-Gly-Ser-Gly-Gly-Gly-Gly-Ser (SEQ ID NO:2). Other examples of linkers include, but are not limited to, Gly-Ser-Gly-Gly-Gly-Gly-Ser-Gly-Gly-Gly-Gly-Ser-Gly-Gly-Gly-Gly-Ser-Gly-Gly-Gly-Gly-Ser (1.5L) (SEQ ID NO:4); Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser (2L) (SEQ ID NO:6); Asp-Ala-Ala-Ala-Lys-Glu-Ala-Ala-Ala-Lys-Asp-Ala-Ala-Ala-Arg-Glu-Ala-Ala-Ala-Arg-Asp-Ala-Ala-Ala-Lys (SEQ ID NO:7) and Asn-Val-Asp-His-Lys-Pro-Ser-Asn-Thr-Lys-Val-Asp-Lys-Arg (SEQ ID NO:8).

The C-terminus of the peptide portion and the N-terminus of the Fc portion are fused together. Alternatively, the N-terminus of the peptide portion and the C-terminus of the Fc portion are fused together. Additionally, the C-terminus of the peptide portion is fused to the N-terminus of the Fc portion and the N-terminus of another peptide molecule is fused to the C-terminus of the Fc portion, resulting in a peptide-Fc-peptide fusion protein.

The present invention also includes polynucleotides encoding the heterologous fusion proteins of the present invention, as well as vectors and host cells comprising such polynucleotides. The heterologous fusion proteins of the present invention may be used to treat patients suffering from human diseases as well as a variety of other conditions or disorders.

The heterologous fusion proteins of the present invention comprise an active therapeutic peptide portion and an Fc portion. The Fc portion comprises substitutions to the human IgG4 sequence that provide the heterologous fusion protein with increase *in vivo* stability compared to the active therapeutic peptide not fused to an Fc sequence.

The heterologous fusion proteins of the present invention contain an Fc portion which is derived from human IgG4, but comprises particular substitutions compared to the wild-type human sequence. As used herein, the Fc portion of an immunoglobulin has the meaning commonly given to the term in the field of immunology. Specifically, this term refers to an antibody fragment which does not contain the two antigen binding regions (the Fab fragments) from the antibody. The Fc portion consists of the constant region of an antibody from both heavy chains, which associate through non-covalent interactions and disulfide bonds. The Fc portion can include the hinge regions and extend through the CH2 and CH3 domains to the c-terminus of the antibody. The Fc portion can further include one or more glycosylation sites.

There are five types of human immunoglobulins with different effector functions and pharmacokinetic properties. IgG is the most stable of the five types having a serum half-life in humans of about 23 days. There are four IgG subclasses (G1, G2, G3, and G4) each of which has different biological functions known as effector functions. These effector functions are generally mediated through interaction with the Fc gamma receptor (FcγR) or by binding a subcomponent of complement 1 (C1q) which recognizes and binds to the heavy chain of Immunoglobulin G or Immunoglobulin M initiating the classical complement pathway. Binding to FcyR can lead to antibody dependent cell mediated cytolysis, whereas binding to complement factors can lead to complement mediated cell lysis. In designing heterologous fusion proteins wherein the Fc portion is being utilized solely for its ability to extend half-life, it is important to minimize any effector function. Thus, the heterologous fusion proteins of the present invention are derived from the human IgG4 Fc region because of its reduced ability to bind FcγR and complement factors compared to other IgG sub-types. IgG4, however, has been shown to deplete target cells in humans [Issacs et al., (1996) Clin. Exp. Immunol. 106:427-433]. Because the heterologous fusion proteins of the present invention target cells in various organs in the body, using an IgG4 derived region in an heterologous fusion protein could initiate an immune response against the cells through interaction of the heterologous fusion protein with receptors present on the target cells. Thus, the IgG4 Fc region which is part of the heterologous fusion proteins of the present invention contains substitutions that eliminate effector function. The IgG4 Fc portion of the heterologous fusion proteins of the present invention contain the following substitutions: alanine or valine for phenylalanine at residue 234 and alanine for leucine at residue 235 (EU numbering, Kabat, E.A. et al. (1991) Sequences of Proteins of Immunological Interest*,* 5^{th} Ed. U.S. Dept. of Health and Human Services, Bethesda, MD, NIH Publication no. 91-3242). In addition, proline may be substituted for glutamate at residue 233. Residues 233, 234 and 235 correspond to positions 16, 17 and 18 in SEQ ID NO: 1. Further, removing the N-linked glycosylation site in the IgG4 Fc region by substituting Ala for Asn at residue 297 (EU numbering) which corresponds to position 80 of SEQ ID NO: is another way to ensure that residual effector activity is eliminated in the context of a heterologous fusion protein.

In addition, the IgG4 Fc portion of the heterologous fusion proteins of the present invention contain a substitution that stabilizes heavy chain dimer formation and prevents the formation of half-IgG4 Fc chains. The heterologous fusion proteins of the present invention preferably exist as_dimers joined together by disulfide bonds and various non-covalent interactions. Wild-type IgG4 contains a Pro-Pro-Cys-Pro-Ser-Cys (SEQ ID NO:3) motif beginning at residue 224 (EU numbering). This motif in a single active therapeutic peptide-Fc chain forms disulfide bonds with the corresponding motif in another active therapeutic peptide-Fc chain. However, the presence of serine in the motif causes the formation of single chain heterologous fusion proteins. The present invention encompasses heterologous fusion proteins wherein the IgG4 sequence is further modified such that serine at position at 228 (EU numbering) is substituted with proline (amino acid residue 11 in SEQ ID NO:1).

The C-terminal lysine residue present in the native molecule may be deleted in the IgG4 derivative Fc portion of the heterologous fusion proteins discussed herein (position 230 of SEQ ID NO:1; deleted lysine referred to as desK). Heterologous fusion proteins expressed in some cell types (such as NSO cells) wherein lysine is encoded by the C-terminal codon are heterogeneous in that a portion of the molecules have lysine as the C-terminal amino acid and a portion have lysine deleted. The deletion is due to protease action during expression in some types of mammalian cells. Thus, to avoid this heterogeneity, it is preferred that heterologous fusion expression constructs lack a C-terminal codon for lysine.

It is preferred that the C-terminal amino acid of the active therapeutic peptide portion is fused to the N-terminus of the IgG4 Fc analog portion via a glycine-rich linker. The *in vivo* function and stability of the heterologous fusion proteins of the present invention can be optimized by adding small peptide linkers to prevent potentially unwanted domain interactions. Further, a glycine-rich linker provides some structural flexibility such that the active therapeutic peptide portion can interact productively with its receptor on target cells. These linkers, however, can significantly increase the risk that the heterologous fusion protein will be immunogenic *in vivo*. Thus, it is preferred that the length be no longer than necessary to prevent unwanted domain interactions and/or optimize biological activity and/or stability. The preferred glycine-rich linker comprises the sequence: Gly-Gly-Gly-Gly-Ser-Gly-Gly-Gly-Gly-Ser-Gly-Gly-Gly-Gly-Ser (SEQ ID NO:2). Although more copies of this linker may be used in the heterologous fusion proteins of the present invention, it is preferred that a single copy of this linker be used to minimize the risk of immunogenicity associated with prolonged and repeated administration.

An active therapeutic peptide can be, without limitation, an enzyme, an enzyme inhibitor, an antigen, an antibody, a hormone, a factor involved in the control of coagulation, an interferon, a cytokine, a growth factor and/or differentiation factor, a factor involved in the genesis/resorption of bone tissues, a factor involved in cellular motility or migration, a bactericidal or antifungal factor, a chemotactic factor, a cytostatic factor, a plasma or interstitial adhesive molecule or extracellular matrices, or alternatively any peptide sequence which is an antagonist or agonist of molecular and/or intercellular interactions involved in the pathologies of the circulatory and interstitial compartments and for example the formation of arterial and venous thrombi, cancerous metastases, tumor angiogenesis, inflammatory shock, autoimmune diseases, bone and osteoarticular pathologies and the like. Examples of active therapeutic peptides include, but are not limited to, G-CSF, GM-CSF, eosinophil (EOS)-CSF, macrophage (M)-CSF, multi-CSF, erythropoietin (EPO), IL-1, IL-2, IL-4, IL-6, IL-7 IL-8, IL-9, IL-10, IL-11, IL-12, IL-13, IL-18, c-kit ligand, fibroblast growth factor (FGF) 21, Stem-cell factor (SCF), mast cell growth factor, erythroid potentiating activity (EPA), Lactoferrin (LF), H- subunit ferritin (i.e., acidic isoferritin), prostaglandin (PG) E1 and E2, tumor necrosis factor (TNF)-α, -β (i.e. lymphotoxin), interferon (IFN)-α (1b 2a and 2b), -β, - ω and -γ; transforming growth factor (TGF)-β, activin, inhibin, leukemic inhibitory factor, oncostatin M, macrophage inflammatory protein (MIP) -1-α (i.e. Stem-cell inhibitor), macrophage inflammatory protein (MIP) -1β, macrophage inflammatory protein (MIP)-2-α (i.e., GRO-β), GRO-α, MIP-2-β (i.e., GRO-γ), platelet factor-4, macrophage chemotactic and activating factor, IP-10, Calcitonin, Growth hormone, PTH, TR6, BLyS, BLyS single chain antibody, Resistin, Growth hormone releasing factor, VEGF-2, KGF-2, D- SLAM, KDI, TR2, Glucagon-like Peptide-1 (GLP-1), Exendin 4, and neuropeptide pituitary adenylate cyclase-activating polypeptide (PACAP), or one of its receptors PAC-1, VPAC-1 or VPAC-2, or active analogs, fragments, or derivatives of any of the before mentioned peptides.

The nomenclature used herein to refer to specific heterologous fusion proteins is defined as follows: L refers to a linker with the sequence Gly-Gly-Gly-Gly-Ser-Gly-Gly-Gly-Gly-Ser-Gly-Gly-Gly-Gly-Ser (SEQ ID NO:2). The number immediately preceding the L refers to the number of linkers separating the active therapeutic peptide portion from the Fc portion. A linker specified as 1.5L refers to the sequence Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:4). A linker specified as 2L refers to the sequence Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:6). IgG4 refers to an analog of the human IgG4 Fc sequence specified as SEQ ID NO:1. Substitutions in the IgG4 Fc portion of the heterologous fusion protein are indicated in parenthesis. The wild-type amino acid is specified by its common abbreviation followed by the position number in the context of the entire IgG4 sequence using the EU numbering system followed by the amino acid being substituted at that position specified by its common abbreviation.

Although the heterologous fusion proteins of the present invention can be made by a variety of different methods, because of the size of the heterologous fusion protein, recombinant methods are preferred. For purposes of the present invention, as disclosed and claimed herein, the following general molecular biology terms and abbreviations are defined below.

"Base pair" or "bp" as used herein refers to DNA or RNA. The abbreviations A,C,G, and T correspond to the 5'-monophosphate forms of the deoxyribonucleosides (deoxy)adenosine, (deoxy)cytidine, (deoxy)guanosine, and thymidine, respectively, when they occur in DNA molecules. The abbreviations U,C,G, and A correspond to the 5'-monophosphate forms of the ribonucleosides uridine, cytidine, guanosine, and adenosine, respectively when they occur in RNA molecules. In double stranded DNA, base pair may refer to a partnership of A with T or C with G. In a DNA/RNA, heteroduplex base pair may refer to a partnership of A with U or C with G. (See the definition of "complementary", infra.)

"Digestion" or "Restriction" of DNA refers to the catalytic cleavage of the DNA with a restriction enzyme that acts only at certain sequences in the DNA ("sequence-specific endonucleases"). The various restriction enzymes used herein are commercially available and their reaction conditions, cofactors, and other requirements were used as would be known to one of ordinary skill in the art. Appropriate buffers and substrate amounts for particular restriction enzymes are specified by the manufacturer or can be readily found in the literature.

"Ligation" refers to the process of forming phosphodiester bonds between two double stranded nucleic acid fragments. Unless otherwise provided, ligation may be accomplished using known buffers and conditions with a DNA ligase, such as T4 DNA ligase.

"Plasmid" refers to an extrachromosomal (usually) self-replicating genetic element.

"Recombinant DNA cloning vector" as used herein refers to any autonomously replicating agent, including, but not limited to, plasmids and phages, comprising a DNA molecule to which one or more additional DNA segments can or have been added.

"Recombinant DNA expression vector" as used herein refers to any recombinant DNA cloning vector in which a promoter to control transcription of the inserted DNA has been incorporated.

"Transcription" refers to the process whereby information contained in a nucleotide sequence of DNA is transferred to a complementary RNA sequence.

"Transfection" refers to the uptake of an expression vector by a host cell whether or not any coding sequences are, in fact, expressed. Numerous methods of transfection are known to the ordinarily skilled artisan, for example, calcium phosphate co-precipitation, liposome transfection, and electroporation. Successful transfection is generally recognized when any indication of the operation of this vector occurs within the host cell.

"Transformation" refers to the introduction of DNA into an organism so that the DNA is replicable, either as an extrachromosomal element or by chromosomal integration. Methods of transforming bacterial and eukaryotic hosts are well known in the art, many of which methods, such as nuclear injection, protoplast fusion or by calcium treatment using calcium chloride are summarized in J. Sambrook, et al., Molecular Cloning: A Laboratory Manual; (1989). Generally, when introducing DNA into Yeast the term transformation is used as opposed to the term transfection.

"Translation" as used herein refers to the process whereby the genetic information of messenger RNA (mRNA) is used to specify and direct the synthesis of a polypeptide chain.

"Vector" refers to a nucleic acid compound used for the transfection and/or transformation of cells in gene manipulation bearing polynucleotide sequences corresponding to appropriate protein molecules which, when combined with appropriate control sequences, confers specific properties on the host cell to be transfected and/or transformed. Plasmids, viruses, and bacteriophage are suitable vectors. Artificial vectors are constructed by cutting and joining DNA molecules from different sources using restriction enzymes and ligases. The term "vector" as used herein includes Recombinant DNA cloning vectors and Recombinant DNA expression vectors.

"Complementary" or "Complementarity", as used herein, refers to pairs of bases (purines and pyrimidines) that associate through hydrogen bonding in a double stranded nucleic acid. The following base pairs are complementary: guanine and cytosine; adenine and thymine; and adenine and uracil.

"Primer" refers to a nucleic acid fragment which functions-as-an initiating substrate for enzymatic or synthetic elongation.

"Promoter" refers to a DNA sequence which directs transcription of DNA to RNA.

"Probe" refers to a nucleic acid compound or a fragment, thereof, which hybridizes with another nucleic acid compound.

"Leader sequence" refers to a sequence of amino acids which can be enzymatically or chemically removed to produce the desired polypeptide of interest.

"Secretion signal sequence" refers to a sequence of amino acids generally present at the N-terminal region of a larger polypeptide functioning to initiate association of that polypeptide with the cell membrane compartments like endoplasmic reticulum and secretion of that polypeptide through the plasma membrane.

Wild-type human IgG4 proteins can be obtained from a variety of sources. For example, these proteins can be obtained from a cDNA library prepared from cells which express the mRNA of interest at a detectable level. Libraries can be screened with probes designed using the published DNA or protein sequence for the particular protein of interest. For example, immunoglobulin light or heavy chain constant regions are described in Adams, et al. (1980) Biochemistry 19:2711-2719; Goughet, et al. (1980) Biochemistry 19:2702-2710; Dolby, et al. (1980) Proc. Natl. Acad. Sci. USA 77:6027-6031; Rice et al. (1982) Proc. Natl. Acad. Sci. USA 79:7862-7862; Falkner, et al. (1982) Nature 298:286-288; and Morrison, et al. (1984) Ann. Rev. Immunol. 2:239-256.

Screening a cDNA or genomic library with the selected probe may be conducted using standard procedures, such as described in Sambrook et al., Molecular Cloning: A Laboratory Manual, Cold Spring Harbor Laboratory Press, NY (1989). An alternative means to isolate a gene encoding an immunoglobulin protein is to use PCR methodology [Sambrook et al., supra; Dieffenbach et al., PCR Primer: A Laboratory Manual, Cold Spring Harbor Laboratory Press, NY (1995)]. PCR primers can be designed based on published sequences.

Generally the full-length wild-type sequences cloned from a particular library can serve as a template to create the IgG4 Fc analog fragments of the present invention that retain the ability to confer a longer plasma half-life on the active therapeutic peptide that is part of the heterologous fusion protein. The IgG4 Fc analog fragments can be generated using PCR techniques with primers designed to hybridize to sequences corresponding to the desired ends of the fragment. PCR primers can also be designed to create restriction enzyme sites to facilitate cloning into expression vectors.

DNA encoding the active therapeutic peptides of the present invention can be made by a variety of different methods including cloning methods like those described above as well as chemically synthesized DNA. Chemical synthesis may be attractive given the short length of the encoded peptide. The amino acid sequence for the active therapeutic peptides are generally known and published [Lopez, et al. (1983) Proc. Natl. Acad. Sci., USA 80:5485-5489; Bell, et al. (1983) Nature, 302:716-718; Heinrich, G., et al. (1984) Endocrinol, 115:2176-2181; Ghiglione, M., et al. (1984) Diabetologia 27:599-600].

The gene encoding a heterologous fusion protein can then be constructed by ligating DNA encoding an active therapeutic protein in-frame to DNA encoding the IgG Fc proteins described herein. The DNA encoding an active therapeutic protein and IgG4 Fc fragments can be mutated either before ligation or in the context of a cDNA encoding an entire heterologous fusion protein. A variety of mutagenesis techniques are well known in the art. The gene encoding the active therapeutic protein and the gene encoding the IgG4 Fc analog protein can also be joined in-frame via DNA encoding a G-rich linker peptide. An example of a DNA sequence encoding one of the heterologous fusion proteins of the present invention, Gly⁸-Glu²²Gly³⁶-GLP-1(7-37)-1L-IgG4 (S228P, F234A, L235A, des K), is provided as SEQ ID NO:5:

Host cells are transfected or transformed with expression or cloning vectors described herein for heterologous fusion protein production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press, 1991) and Sambrook, *et al.,* supra. Methods of transfection are known to the ordinarily skilled artisan, for example, CaPO₄ and electroporation. General aspects of mammalian cell host system transformations have been described in U.S. Patent No. 4,399,216. Transformations into yeast are typically carried out according to the method of van Solingen et al., J Bact. 130(2): 946-7 (1977) and Hsiao et al., Proc. Natl. Acad. Sci. USA 76(8): 3829-33 (1979). However, other methods for introducing DNA into cells, such as by nuclear microinjection, electroporation, bacterial protoplast fusion with intact cells, or polycations, e.g., polybrene or polyomithine, may also be used. For various techniques for transforming mammalian cells, see Keown, et al., Methods in Enzymology 185: 527-37 (1990) and Mansour, et al., Nature 336(6197): 348-52 (1988).

Suitable host cells for cloning or expressing the nucleic acid (e.g., DNA) in the vectors herein include yeast or higher eukaryote cells.

Eukaryotic microbes such as filamentous fungi or yeast are suitable cloning or expression hosts for heterologous fusion protein vectors. Saccharomyces cerevisiae is a commonly used lower eukaryotic host microorganism. Others include Schizosaccharomyces pombe [Beach and Nurse, Nature 290: 140-3 (1981); EP 139,383 published 2 May 1995]; Muyveromyces hosts [U.S. Patent No. 4,943,529; Fleer, et al., Bio/Technology 9(10): 968-75 (1991)] such as, e.g., K lactis (MW98-8C, CBS683, CBS4574) [de Louvencourt et al., J. Bacteriol. 154(2): 737-42 (1983)]; K. fiagilis (ATCC 12,424), K. bulgaricus (ATCC 16,045), K wickeramii (ATCC 24,178), K waltii (ATCC 56,500), K. drosophilarum (ATCC 36.906) [Van den Berg et al., BiolTechnology 8(2): 135-9 (1990)]; K. thermotoierans, and K. marxianus; yarrowia (EP 402,226); Pichia pastoris (EP 183,070) [Sreekrishna et al., J. Basic Microbiol. 28(4): 265-78 (1988)]; Candid; Trichoderma reesia (EP 244,234); Neurospora crassa [Case, et al., Proc. Natl. Acad Sci. USA 76(10): 5259-63 (1979)]; Schwanniomyces such as Schwanniomyces occidentulis (EP 394,538 published 31 October 1990); and filamentous fungi such as, e.g., Neurospora, Penicillium, Tolypocladium (WO 91/00357 published 10 January 1991), and Aspergillus hosts such as A. nidulans [Ballance et al., Biochem. Biophys. Res. Comm. 112(1): 284-9 (1983)]; Tilburn, et al., Gene 26(2-3): 205-21 (1983); Yelton, et al., Proc. Natl. Acad. Sci. USA 81(5): 1470-4 (1984)] and A. niger [Kelly and Hynes, EMBO J. 4(2): 475-9 (1985)]. Methylotropic yeasts are selected from the genera consisting of Hansenula, Candida, Kloeckera, Pichia, Saccharomyces, Torulopsis, and Rhodotoruia. A list of specific species that are exemplary of this class of yeast may be found in C. Antony, The Biochemistry of Methylotrophs 269 (1982).

Suitable host cells for the expression of the heterologous fusion proteins of the present invention are derived from multicellular organisms. Examples of invertebrate cells include insect cells such as Drosophila S2 and Spodoptera Sp, Spodoptera high5 as well as plant cells. Examples of useful mammalian host cell lines include NSO myeloma cells, Chinese hamster ovary (CHO), SP2, and COS cells. More specific examples include monkey kidney CV1 line transformed by SV40 (COS-7, ATCC CRL 1651); human embryonic kidney line [293 or 293 cells subcloned for growth in suspension culture, Graham, et al., J. Gen Virol., 36(1): 59-74 (1977)]; Chinese hamster ovary cells/-DHFR [CHO, Urlaub and Chasin, Proc. Natl. Acad. Sci. USA, 77(7): 4216-20 (1980)]; mouse sertoli cells [TM4, Mather, Biol. Reprod. 23(1):243-52 (1980)]; human lung cells (W138. ATCC CCL 75); human liver cells (Hep G2, HB 8065); and mouse mammary tumor (MMT 060562, ATCC CCL51). A preferred cell line for production of the heterologous fusion proteins of the present invention is the *NS0* myeloma cell line available from the European Collection of Cell Cultures (ECACC, catalog #85110503) and described in Galfre, G. and Milstein, C. ((1981) Methods in Enzymology 73(13):3-46; and Preparation of Monoclonal Antibodies: Strategies and Procedures, Academic Press, N.Y., N.Y.).

The heterologous fusion proteins of the present invention may be recombinantly produced directly, or as a protein having a signal sequence or other additional sequences which create a specific cleavage site at the N-terminus of the mature heterologous fusion protein. In general, the signal sequence may be a component of the vector, or it may be a part of the heterologous fusion protein-encoding DNA that is inserted into the vector. For yeast secretion the signal sequence may be, e.g., the yeast invertase leader, alpha factor leader (including Saccharomyces and Kluyveromyces cc-factor leaders, the latter described in U.S. Patent No. 5,010,182), or acid phosphatase leader, the C. albicans glucoamylase leader (EP 362,179), or the signal described in WO 90/13646. In mammalian cell expression, mammalian signal sequences may be used to direct secretion of the protein, such as signal sequences from secreted polypeptides of the same or related species as well as viral secretory leaders.

Both expression and cloning vectors contain a nucleic acid sequence that enables the vector to replicate in one or more selected host cells. Expression and cloning vectors will typically contain a selection gene, also termed a selectable marker. Typical selection genes encode proteins that (a) confer resistance to antibiotics or other toxins, e.g., neomycin, methotrexate, or tetracycline, (b) complement autotrophic deficiencies, or (c) supply critical nutrients not available from complex media, e.g., the gene encoding D-alanine racemase for Bacilli.

An example of suitable selectable markers for mammalian cells are those that enable the identification of cells competent to take up the heterologous fusion protein-encoding nucleic acid, such as DHFR or thymidine kinase. An appropriate host cell when wild-type DHFR is employed is the CHO cell line deficient in DHFR activity, prepared and propagated as described [Urlaub and Chasin, Proc. Natl. Acad. Sci. USA, 77(7): 4216-20 (1980)]. A suitable selection gene for use in yeast is the trpl gene present in the yeast plasmid YRp7 [Stinchcomb, et al., Nature 282(5734): 39-43 (1979); Kingsman, et al., Gene 7(2): 141-52 (1979); Tschumper, et al., Gene 10(2): 157-66 (1980)]. The trpl gene provides a selection marker for a mutant strain of yeast lacking the ability to grow in tryptophan, for example, ATCC No. 44076 or PEPC1 [Jones, Genetics 85: 23-33 (1977)].

Expression and cloning vectors usually contain a promoter operably linked to the heterologous fusion protein-encoding nucleic acid sequence to direct mRNA synthesis. Promoters recognized by a variety of potential host cells are well known. Examples of suitable promoting sequences for use with yeast hosts include the promoters for 3-phosphoglycerate kinase [Hitzeman, et al., J. Biol. Chem. 255(24): 12073-80 (1980)] or other glycolytic enzymes [Hess et al., J. Adv. Enzyme Reg. 7: 149 (1968); Holland, Biochemistry 17(23): 4900-7 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Transcription of heterologous fusion protein-encoding mRNA from vectors in mammalian host cells may be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a polynucleotide encoding a heterologous fusion protein by higher eukaryotes may be increased by inserting an enhancer sequence into the vector. Enhancers are cis acting elements of DNA, usually about from 10 to 300 bp, that act on a promoter to increase its transcription. Many enhancer sequences are now known from mammalian genes (globin, elastase, albumin, a-ketoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the heterologous fusion protein coding sequence but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and occasionally 3' untranslated regions of eukaryotic or viral DNAs or cDNAs. These regions contain nucleotide segments transcribed as polyadenylated fragments in the untranslated portion of the mRNA encoding the heterologous fusion protein.

Various forms of a heterologous fusion protein may be recovered from culture medium or from host cell lysates. If membrane-bound, it can be released from the membrane using a suitable detergent solution (e.g., Triton-X 100) or by enzymatic cleavage. Cells employed in expression of a heterologous fusion protein can be disrupted by various physical or chemical means, such as freeze-thaw cycling, sonication, mechanical disruption, or cell lysing agents.

Once the heterologous fusion proteins of the present invention are expressed in the appropriate host cell, the analogs can be isolated and purified. The following procedures are exemplary of suitable purification procedures: fractionation on carboxymethyl cellulose; gel filtration such as Sephadex G-75; anion exchange resin such as DEAE or Mono-Q; cation exchange such as CM or Mono-S; metal chelating columns to bind epitope-tagged forms of the polypeptide; reversed-phase HPLC; chromatofocusing; silica gel; ethanol precipitation; and ammonium sulfate precipitation.

Various methods of protein purification may be employed and such methods are known in the art and described, for example, in Deutscher, Methods in Enzymology 182: 83-9 (1990) and Scopes, Protein Purification: Principles and Practice, Springer-Verlag, NY (1982). The purification step(s) selected will depend on the nature of the production process used and the particular heterologous fusion protein produced. For example, heterologous fusion proteins comprising an Fc fragment can be effectively purified using a Protein A or Protein G affinity matrix. Low or high pH buffers can be used to elute the heterologous fusion protein from the affinity matrix. Mild elution conditions will aid in preventing irreversible denaturation of the heterologous fusion protein.

The heterologous fusion proteins of the present invention may be formulated with one or more excipients. The heterologous fusion proteins of the present invention may be combined with a pharmaceutically acceptable buffer, and the pH adjusted to provide acceptable stability, and a pH acceptable for administration such as parenteral administration. Optionally, one or more pharmaceutically-acceptable anti-microbial agents may be added. Meta-cresol and phenol are preferred pharmaceutically-acceptable microbial agents. One or more pharmaceutically-acceptable salts may be added to adjust the ionic strength or tonicity. One or more excipients may be added to further adjust the isotonicity of the formulation. Glycerin is an example of an isotonicity-adjusting excipient. Pharmaceutically acceptable means suitable for administration to a human or other animal and thus, does not contain toxic elements or undesirable contaminants and does not interfere with the activity of the active compounds therein.

The heterologous fusion proteins of the present invention may be formulated as a solution formulation or as a lyophilized powder that can be reconstituted with an appropriate diluent. A lyophilized dosage form is one in which the heterologous fusion protein is stable, with or without buffering capacity to maintain the pH of the solution over the intended in-use shelf-life of the reconstituted product. It is preferable that the solution comprising the heterologous fusion proteins discussed herein before lyphilization be substantially isotonic to enable formation of isotonic solutions after reconstitution.

A pharmaceutically-acceptable salt form of the heterologous fusion proteins of the present invention are within the scope of the invention. Acids commonly employed to form acid addition salts are inorganic acids such as hydrochloric acid, hydrobromic acid, hydriodic acid, sulfuric acid, phosphoric acid, and the like, and organic acids such as *p*-toluenesulfonic acid, methanesulfonic acid, oxalic acid, *p*-broinophenyl-sulfonic acid, carbonic acid, succinic acid, citric acid, benzoic acid, acetic acid, and the like. Preferred acid addition salts are those formed with mineral acids such as hydrochloric acid and hydrobromic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like.

The heterologous fusion proteins of the present invention have biological activity. Biological activity refers to the ability of the heterologous fusion protein to bind to and activate a receptor *in vivo* and elicit a response. A representative number of heterologous fusion proteins were tested for *in vitro* as well as *in vivo* activity. Examples 1 and 2 provide a representation of *in vitro* activity based on the ability of the heterologous fusion protein to interact with and activate the human GLP-1 receptor. In both sets of experiments, HEK293 cells over-expressing the human GLP-1 receptor are used. Activation of the GLP-1 receptor in these cells causes adenylyl cyclase activation which in turn induces expression of a reporter gene driven by a cyclic AMP response element (CRE). Example 1 (table 1) provides representative data wherein the reporter gene is beta lactamase, and example 2 (table 2) provides representative data wherein the reporter gene is luciferase. Example 3 provides representative data generated after administration of a heterologous fusion proteins of the present invention to rats. Example 4 (table 6) provides representative data generated after administration of a heterologous fusion proteins of the present invention to monkeys.
Example 5 (table 7) provides representative data of the assessment of the potential formation of antibodies following repeat subcutanesous injections of a heterologous fusion protein. Example 6 (table 8) provides representative data from a pharmacodynamic study following an injection of a heterologous fusion protein to monkey. Example 7 (table 9) provides representative data from a pharmacodynamic study following injections of three different doses to rats. Example 8 (table 10) provides representative data generated after administration of a different heterologous fusion proteins of the present invention to mice. Together the representative data show that the heterologous fusion proteins are able to bind to and activate their receptor, appear more potent than the active therapeutic peptide, are active *in vivo* and have a longer half-life than the active therapeutic peptide, are not immunogenic, and are dose responsive.

Administration of the heterologous fusion proteins may be via any route known to be effective by the physician of ordinary skill. Peripheral parenteral is one such method. Parenteral administration is commonly understood in the medical literature as the injection of a dosage form into the body by a sterile syringe or some other mechanical device such as an infusion pump. Peripheral parenteral routes can include intravenous, intramuscular, subcutaneous, and intraperitoneal routes of administration.

The heterologous fusion proteins of the present invention may also be amenable to administration by oral, rectal, nasal, or lower respiratory routes, which are non-parenteral routes. Of these non-parenteral routes, the lower respiratory route and the oral route are preferred.

The heterologous fusion proteins of the present invention can be used to treat a wide variety of diseases and conditions.

An effective amount of the heterologous fusion proteins described herein is the quantity which results in a desired therapeutic and/or prophylactic effect without causing unacceptable side-effects when administered to a subject in need of the active therapeutic peptide receptor stimulation. A "desired therapeutic effect" includes one or more of the following: 1) an amelioration of the symptom(s) associated with the disease or condition; 2) a delay in the onset of symptoms associated with the disease or condition; 3) increased longevity compared with the absence of the treatment; and 4) greater quality of life compared with the absence of the treatment.

It is preferable that the heterologous fusion proteins of the present invention be administered either once every two weeks or once a week. Depending on the disease being treated, it may be necessary to administer the heterologous fusion protein more frequently such as two to three time per week.

The present invention will now be described only by way of non-limiting example with reference to the following Examples.

### EXAMPLES

### Example 1 - In vitro GLP-1 receptor activation assay

HEK-293 cells expressing the human GLP-1 receptor, using a CRE-BLAM system, are seeded at 20,000 to 40,000 cells/well/100 µl DMEM medium with 10%FBS into a poly-d-lysine coated 96 well black, clear-bottom plate. The day after seeding, the medium is flicked off and 80 µl plasma free DMEM medium is added. On the third day after seeding, 20 µl of plasma-free DMEM medium with 0.5% BSA containing different concentrations of various GLP-1-Fc heterologous fusion protein is added to each well to generate a dose response curve. Generally, fourteen dilutions containing from 3 nanomolar to 30 nanomolar or heterologous GLP-1 Fc fusion protein are used to generate a dose response curve from which EC₅₀ values can be determined. After 5 hours of incubation with the fusion protein, 20 µl of β-lactamase substrate (CCF2/AM, PanVera LLC) is added and incubation continued for 1 hour at which time fluorescence is determined on a cytofluor. The assay is further described in Zlokarnik, et al. (1998), Science, 278:84-88. Various GLP-1-Fe fusion proteins are tested and EC₅₀ values are represented in Table 1. The values are relative to values determined for Val⁸-GLP-1(7-37)OH which is run as an internal control with every experiment.

**Table 1**

| Compound | Activity | Std. Dev. |
|---|---|---|
| Val⁸-GLP-1: 1 : | 100% | |
| Gly⁸-Glu²²-GLP-1(7-37)-2L-IgG4 (S228P, F234A, L235A): | 301% | 99 |
| Gly⁸-G1u²²-GLP-1(7-37)-1.5L-IgG4 (S228P, F234A, L235A): | 314% | 45 |
| Gly⁸-Glu²²-GLP-1(7-37)-1L-IgG4 (S228P, F234A, L235A): | 468% | 120 |
| Gly⁸-Glu²²-Gly³⁶-GLP-1(7-37)-2L-IgG4 (S228P, F234A, L235A): | 441% | 35 |

### Example 2 - In vitro GLP-1 receptor activation assay

HEK-293 cells stably expressing the human GLP-1 receptor, using a CRE-Luciferase system, are seeded at 30,000 cells/well/80 µl low serum DMEM F12 medium into 96 well plates. The day after seeding, 20 µl aliquots of test protein dissolved in 0.5% BSA are mixed and incubated with the cells for 5 hours. Generally 12 dilutions containing from 3 pM to 3 nM are prepared at a 5X concentration for each test protein before addition to the cells to generate a dose response curve from which EC₅₀ values are determined. After incubation, 100 µl of Luciferase reagent is added directly to each plate and mixed gently for 2 minutes. Plates are placed in a Tri-lux luminometer and light output resulting from luciferase expression is calculated. Various GLP-1-Fc fusion proteins are tested and EC₅₀ values are represented in Table 2. The values are relative to values determined for Val⁸-GLP-1(7-37)OH which is run as an internal control with every experiment. Because the heterologous fusion proteins tested below are dimers, values are corrected taking into account a 2-fold difference in molarity.

**Table 2**

| Compound | Activity Std. Dev. |
|---|---|
| Val⁸-GLP-1 : | 100% |
| Gly⁸-Glu²²-GLP-1(7-37)-2L-IgG4 (S228P, F234A, L235A): | 535% 240 |
| Gly⁸-G1u²²-GLP-1(7-37)-1.5L-IgG4 (S228P, F234A, L235A): | 595% 43 |
| Gly⁸-Glu²²-GLP-1(7-37)-1L-IgG4 (S228P, F234A, L235A): | 1119% 128 |
| Gly⁸-Glu²²-Gly³⁶-GLP-1(7-37)-2L-IgG4 (S228P, F234A, L235A): | 398% 62 |
| Gly⁸-Glu²²-Gly³⁶-GLP-1(7-37)-1L-IgG4 (S228P, F234A, L235A): | 417% 140 |

### Example 3 Intravenous Glucose Tolerance Test in Rats

The heterologous fusion protein, Gly⁸-Glu²²-Gly³⁶-GLP-1(7-37)-L-IgG4 (S228P,F234A,L235A), is evaluated in an intravenous glucose tolerance test (IVGTT) in rats. At least four rats are included into each of three groups. Group I receives vehicle (table 3), Group II receives 1.79 mg/kg of Gly⁸-Glu²²-Gly³⁶-GLP-1(7-37)-L-IgG4 (S228P,F234A;L235A) as a single subcutaneous injection (table 4), and Group III receives 0.179 mg/kg of Gly⁸-Glu²²-Gly³⁶-GLP-1(7-37)-L-IgG4 (S228P,F234A,L235A) as a single subcutaneous injection (table 5). Rats are subcutaneously injected the morning of Day 1. Twenty-four hours following the first injection, 1 µL of glucose (D50) per gram rat body weight is infused as a bolus. Blood samples are taken at 2, 4, 6, 10, 20, and 30 minutes following the bolus infusion of glucose.

**Table 3**

| **Vehicle:** | Rat 1 | Rat 2 | Rat 3 | Rat 4 | Rat 5 | | |
|---|---|---|---|---|---|---|---|
| *Insulin AUC (ng*minlmL)* | | | | | | **Average** | **SEM** |
| **0-2** | 11 | 9.4 | 7 | 11 | 9.6 | | |
| **2-4** | 18.1 | 9.7 | 5.6 | 10.6 | 8.8 | | |
| **4-6** | 13.4 | 7 | 3.4 | 9.6 | 5.9 | | |
| **6-10** | 7.9 | 3.5 | 2.5 | 6 | 2.9 | | |
| **10-20** | 3.7 | 3 | 2.4 | 3 | 2.4 | | |
| **20-30** | 2 | 0 | 0 | 0 | 2.4 | | |
| sum | 56.1 | 32.6 | 20.9 | 40.2 | 32 | 36.4 | 5.8 |

**Table 4**

| **GLP-1-Fc** | | | | | | | |
|---|---|---|---|---|---|---|---|
| (1.79mg/kg) | Rat 1 | Rat 2 | Rat 3 | Rat 4 | Rat 5 | | |
| *Insulin AUC (ng*min*/*mL)* | | | | | | **Average** | **SEM** |
| **0-2** | 12.3 | 17.4 | 16 | 14 | 13 | | |
| **2-4** | 21.9 | 13.3 | 13.2 | 13.9 | 13.6 | | |
| **4-6** | 16.8 | 6.5 | 9.8 | 11.1 | 11.7 | | |
| **6-10** | 7.6 | 3.8 | 9.2 | 5.8 | 7.4 | | |
| **10-20** | 3 | 0 | 0 | 3.2 | 5.6 | | |
| **20-30** | 0 | 0 | 0 | 0 | 0 | | |
| **sum** | 61.6 | 41 | 48.2 | 48 | 51.3 | 50 | 3.4 |

**Table 5**

| **GLP-1-Fc** | | | | | | |
|---|---|---|---|---|---|---|
| (0.179mg/kg) | Rat 1 | Rat 2 | Rat 3 | Rat 4 | | |
| *Insulin AUC (ng*min*/*mL)* | | | | | **Average** | **SEM** |
| **0-2** | 14.4 | 29.2 | 25.4 | 23.2 | | |
| **2-4** | 13.8 | 26.3 | 21.2 | 21.8 | | |
| **4-6** | 11.2 | 19.4 | 16.4 | 15.7 | | |
| **6-10** | 6.4 | 10.6 | 10.5 | 8 | | |
| **10-20** | 3.6 | 5.8 | 5.2 | 5 | | |
| **20-30** | 0 | 0 | 0 | 0 | | |
| **sum** | 49.4 | 91.3 | 78.7 | 73.7 | 78.7 | 8.7 |

### Example 4 Pharmacokinetic Study Following a Single Subcutaneous Injection to Cynomolgus Monkeys.

A study is performed to characterize the pharmacokinetics (PK) of the heterologous fusion protein, Gly⁸-Glu²²-Gly³⁶-GLP-1(7-37)-L-IgG4 (S228P,F234A,L235A), when administered as a 0.1 mg/kg by subcutaneous (SC) injection to male cynomolgus monkeys. RIA antibody is specific for the middle portion of GLP. ELISA uses an N-terminus specific capture antibody and an Fc specific detection antibody.
Resulting plasma concentrations from both the ELISA and the RIA are used to determine the represented pharmacokinetic parameter values.

A representation of the resulting PK parameter values is summarized in table 6. Single-dose SC PK from the RIA is associated with a mean Cₘₐₓ of 446.7 ng/mL with a corresponding Tₘₐₓ of 17.3 hours. The mean elimination half-life is approximately 79.3 hours (3.3 days). The PK from the ELISA is associated with a mean Cₘₐₓ of 292.2 ng/mL with a corresponding Tₘₐₓ of 16.7 hours. The mean elimination half-life is approximately 51.6 hours (2.2 days).

**Table 6**

| RIA | | | | | | | |
|---|---|---|---|---|---|---|---|
| Dose | Animal # | Cₘₐₓ^{a} | Tₘₐₓ^{b} | AUC_{0-∞}^{c} | t_{1/2}^{d} | CL/F^{e} | Vss/F^{f} |
| (mg/kg) | | (ng/mL) | (h) | (ng*h/mL) | (h) | (mL/h/kg) | (mL/kg) |
| 0.1 | 96051 | 461.0 | 4.0 | 37770.5 | 81.0 | 2.7 | 309.2 |
| | 96071 | 430.0 | 24.0 | 43150.2 | 74.2 | 2.3 | 248.1 |
| | 96091 | 449.0 | 24.0 | 62271.1 | 82.9 | 1.6 | 191.9 |
| RIA | Mean | 446.7 | 17.3 | 47730.6 | 79.3 | 2.2 | 249.8 |
| | SD | 15.6 | 11.5 | 12876.5 | 4.5 | 0.5 | 58.7 |
| ELISA | | | | | | | |
| | 96051 | 315.4 | 2.0 | 9062.3 | 55.2 | 11.0 | 879.4 |
| | 96071 | 289.4 | 24.0 | 16653.0 | 50.3 | 6.0 | 436.0 |
| | 96091 | 271.9 | 24.0 | 19907.4 | 49.3 | 5.0 | 357.0 |
| ELISA | Mean | 292.2 | 16.7 | 15207.6 | 51.6 | 7.3 | 557.5 |
| | SD | 21.9 | 12.7 | 5565.2 | 3.2 | 3.2 | 281.6 |

| | | | | | | | |
|---|---|---|---|---|---|---|---|
| ^{a} Maximum observed plasma concentration. ^{b} Time of maximum observed plasma concentration. ^{c} Area under the plasma concentration-time curve measured from 0 to infinity. ^{d} Elimination half-life. ^{e} Total body clearance as a function of bioavailability. ^{f} Volume of distribution as a function of bioavailability. SD = Standard deviation. | | | | | | | |

### Example 5 Assessment of the potential formation of antibodies following repeat subcutanesous injections.

Designated serum samples from cynomolgus monkeys are tested for the formation of antibodies against Gly⁸-Glu²²-Gly³⁶-GLP-1(7-37)-L-IgG4 (S228P,F234A,L235A) using a direct ELISA format.. Microtiter plates are coated with Gly⁸-Glu²²-Gly³⁶-GLP-1(7-37)-L-IgG4 (S228P,F234A,L235A) at a 0.1 µg/mL concentration. Monkey serum samples are diluted 50, 500,1000 and 5000 fold into blocking solution, and 0.05 mL sample/well are incubated approximately one hour. Secondary antibody, Goat <Human Fab'2>-Peroxidase (with 75% cross reactivity to human), is diluted 10,000 fold into block and added at 0.05 mL/well and incubated approximately one hour. Color development using tetramethylbenzidine (TMB) substrate is read at an optical density of 450nm - 630nm. Duplicate readings are averaged. A GLP-1 antibody was used as a positive control and goat<rabbit>(H+L)-Peroxidase conjugate is the secondary used for detection. Point serum samples are collected prior to dosing, at 24 hours following the second dose, and 168 hours following the first and second SC dose for an evaluation of potential immunogenicity. The presence of antibody titers to G8E22-CEX-L-hIgG4 is interpreted by comparison to predose serum samples and positive control. A representation of the results is presented in table 7.

**Table 7**

| **Dose 1 Animal#** | **Positive Control** | **IO7774** | | **IO7777** | | **107779** | | **IO7780** | |
|---|---|---|---|---|---|---|---|---|---|
| **Sample Time:** | | **Predose** | **168 h** | **Predose** | **168 h** | **Predose** | **168 h** | **Predose** | **168 h** |
| **50x** | 2.854 | 0.268 | 0.268 | 0.160 | 0.128 | 0.144 | 0.152 | 0.264 | 0.224 |
| **500x** | 2.270 | 0.117 | 0.133 | 0.052 | 0.069 | 0.065 | 0.061 | 0.067 | 0.061 |
| **1000x** | 1.610 | 0.091 | 0.075 | 0.034 | 0.051 | 0.047 | 0.045 | 0.138 | 0.049 |
| **5000x** | 0.525 | 0.056 | 0.048 | 0.032 | 0.037 | 0.029 | 0.033 | 0.051 | 0.039 |
| | | | | | | | | | |

| **Dose 2 Animal#** | **Positive Control** | **IO7774** | | **IO7777** | | **IO7779** | | **IO7780** | |
|---|---|---|---|---|---|---|---|---|---|
| **Sample Time:** | | **Predose** | **24 h** | **Predose** | **24 h** | **Predose** | **24 h** | **Predose** | **24 h** |
| **50x** | 3.056 | 0.298 | 0.231 | 0.164 | 0.159 | 0.227 | 0.176 | 0.211 | 0.192 |
| **500x** | 2.247 | 0.120 | 0.119 | 0.048 | 0.045 | 0.061 | 0.060 | 0.056 | 0.057 |
| **1000x** | 1.673 | 0.090 | 0.086 | 0.039 | 0.041 | 0.046 | 0.045 | 0.043 | 0.048 |
| **5000x** | 0.534 | 0.039 | 0.042 | 0.030 | 0.034 | 0.033 | 0.036 | 0.033 | 0.034 |
| | | | | | | | | | |

| **Dose 2 Animal#** | **Positive Control** | **107774** | | **107777** | | **107779** | | **107780** | |
|---|---|---|---|---|---|---|---|---|---|
| **Sample Time:** | | **Predose** | **168 h** | **Predose** | **168 h** | **Predose** | **168 h** | **Predose** | **168 h** |
| **50x** | 3.075 | 0.413 | 0.270 | 0.174 | 0.182 | 0.185 | 0.190 | 0.224 | 0.191 |
| **500x** | 2.173 | 0.097 | 0.103 | 0.042 | 0.051 | 0.056 | 0.057 | 0.048 | 0.053 |
| **1000x** | 1.510 | 0.066 | 0.067 | 0.038 | 0.040 | 0.037 | 0.046 | 0.043 | 0.043 |
| **5000x** | 0.474 | 0.042 | 0.042 | 0.033 | 0.046 | 0.033 | 0.033 | 0.036 | 0.041 |

### Example 6 Pharmacodynamic Study Following a Single Subcutaneously Injection to Cynomolgus Monkeys in the Fasting State and During a Graded Intravenous Glucose Infusion.

In Phase 1 (Study Day 1) a subcutaneous injection of vehicle is administered. A graded intravenous glucose (20% dextrose) infusion of 5, 10, and 25 mg/kg/min is then administered immediately after the vehicle injection. In Phase 2 (Study Day 3), a subcutaneous injection of a GLP-1 fusion protein (0.1 mg/kg) is administered. In Phase 3, a graded intravenous glucose infusion is performed approximately 96 hours following the GLP-1 fusion injection.

Graded intravenous glucose infusion procedures are conducted in sedated monkeys after a 16-hr overnight fast. For both intravenous glucose infusions, baseline samples will be drawn every 10 min for 20 min to define baseline. A stepped-up glucose infusion is initiated at +20 min at a rate of 5 mg/kg/min, followed by infusions of 10 mg/kg/min, and 25 mg/kg/min. Each infusion rate is administered for a period of 20 minutes. Blood samples are taken at 10 minute intervals for measurement of glucose, insulin, and glucagon. Approximately 1.0 mL of blood is collected at -20, -10 min, 0 pre-glucose infusions, and at 10, 20, 30, 40, 50, and 60 minutes following glucose infusion for Phases 1 and 3.

A representation of the data are shown in table 8.

**Table 8**

| **Glucose AUC** | | | | | |
|---|---|---|---|---|---|
| Group | Animal | AUC (min*mg/dL) | Group | Animal | AUC (min*mg/dL) |
| GLP-Fc | 9423 | 7447 | vehicle | 9423 | 8077 |
| | 9424 | 7470 | | 9424 | 15006 |
| | 9510 | 5153 | | 9510 | 7116 |
| | 9513 | 6303 | | 9513 | 7459 |
| | 9516 | 5413 | | 9516 | 8728 |
| | 9530 | 5240 | | 9530 N | 7863 6 |
| | Mean | 6171 | | Mean | 9041 |
| | SD | 1078 | | SD | 2973 |
| | SE | 440 | | SE | 1214 |

| **Insulin AUC** | | | | | |
|---|---|---|---|---|---|
| Group | Animal | AUC (min*ng/mL) | Group | Animal | AUC (min*ng/mL) |
| GLP-Fc | 9423 | 129 | vehicle | 9423 | 38 |
| | 9424 | 138 | | 9424 | 29 |
| | 9510 | 357 | | 9510 | 69 |
| | 9513 | 161 | | 9513 | 64 |
| | 9516 | 376 | | 9516 | 38 |
| | 9530 | 215 | | 9530 | 68 |
| | Mean | 229 | | Mean | 51 |
| | SD | 111 | | SD | 18 |
| | SE | 45 | | SE | 7 |

| | | | | | |
|---|---|---|---|---|---|
| Glucagon levels were not statistically different between the vehicle and the GLP-1 fusion protein dosed monkeys. | | | | | |

### Example 7 Pharmacodynamic Study Following Single Subcutaneously Injections of Three Different Doses to Rats in the Fasting State and During a Graded Intravenous Glucose Infusion.

Chronically cannulated rats are assigned to either vehicle control (saline) or one of 3 treatment groups (GLP-1 fusion protein; 0.0179 mg/kg, 0.179 mg/kg, or 1.79 mg/kg). The GLP-1 fusion protein and vehicle are administered via subcutaneous injection. Twenty-four hours after treatment, overnight fasted (16h) rats are subjected to a graded intravenous glucose infusion test. The graded glucose infusion test consists of a baseline saline infusion period (20 min), followed by two 30 min glucose infusion phases at 5 and 15 mg/kg/min, respectively. Plasma samples are collected at -20, -10 min, 0 pre-glucose infusions (baseline), and at 10, 20, 30, 40, 50, and 60 minutes.
A representation of the data are shown in table 9.

**Table 9**

| | 5mg/Kg/min | 15mg/Kg/min |
|---|---|---|
| Vehicle | 4.3 ± 0.2 (n=18) | 12.7 ± 0.9 (n=18) |
| 0.0179 mg/Kg | 5.6 ± 0.4 (n=4) | 15.9 ± 1.8 (n=4) |
| 0.179 mg/Kg | 9.0 ± 1.1* (n=6) | 28.0 ± 3.8* (n=6) |
| 1.79 mg/Kg | 20.5 ± 3.0 * (n=4) | 52.7 ± 7.2* (n=4) |

| | | |
|---|---|---|
| *P ≤ 0.05 versus vehicle | | |

### Example 8 Pharmacokinetic analysis of FGF-21 Fusion Protein

FGF-21 fusion proteins are administered by intravenous (IV) or subcutaneous (SC) routes at a dose of 0.4 mg/kg to CD-1 mice. The animals are bled at various times between 0 and 336 hours after dosing. Plasma is collected from each sample and analyzed by radioimmunoassay. Pharmacokinetic parameters are calculated using model-dependent (IV data) and independent (SC data) methods (WinNonlin Pro) and are reported in table 10 below. By IV administration, the FGF-21-Fc fusion protein has an elimination half-life of approximately 53.9 hours compared to an elimination half-life of 0.5 hours for native FGF-21. By SC administration the FGF-21-Fc fusion protein has an elimination half-life of approximately 24 hours compared to an elimination half-life of 0.6 hours for native FGF-21. By both routes of administration the FGF-21-Fc fusion protein demonstrates prolonged time action when compared to native FGF-21.

**Table 10**

| Compound | Route | Cₘₐₓ^{a} (ng/mL) | Tₘₐₓ^{b} (d) | AUC_{0-∞}^{c} (ng*h/mL) | t_{1/2}^{d} (h) | CL/F^{e} (mL/h/kg) | %F^{g} |
|---|---|---|---|---|---|---|---|
| FGF-21-Fc | IV | 4432 | - | 137383 | 53.9 | 2.9 | |
| | SC | 1899 | 24 | 145056 | 48.6 | 2.8 | 106 |
| FGF-21 | IV | 4300 | - | 1200 | 0.5 | 803 | - |
| | SC | 440 | 1.0 | 980 | 0.6 | 1024 | 78 |

| | | | | | | | |
|---|---|---|---|---|---|---|---|
| ^{a} Maximum observed plasma concentration. ^{b} Time of maximum observed plasma concentration. ^{c} Area under the plasma concentration-time curve measured from 0 to infinity. ^{d} Elimination half-life in hours. ^{e} Total body clearance as a function of bioavailability. ^{f} Percent bioavailability. | | | | | | | |

## Claims

1. A heterologous fusion protein comprising an active therapeutic peptide fused to the Fc portion of an immunoglobulin, the Fc portion comprising the sequence of SEQ ID NO: 1 wherein:
Xaa at position 1 is Ala or absent;
Xaa at position 16 is Pro or Glu;
Xaa at position 17 is Val, or Ala;
Xaa at position 18 is Ala;
Xaa at position 80 is Asn or Ala; and
Xaa at position 230 is Lys or is absent.

2. The heterologous fusion protein of claim 1 wherein the C-terminal amino acid of the active therapeutic peptide is fused to the N-terminal residue of the Fc portion via a peptide linker comprising a sequence selected from:
a) Gly-Gly-Gly-Gly-Ser-Gly-Gly-Gly-Gly-Ser-Gly-Gly-Gly-Gly-Ser (SEQ ID NO:2);
b) Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:4);
c) Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:6);
d) Asp-Ala-Ala-Ala-Lys-Glu-Ala-Ala-Ala-Lys-Asp-Ala-Ala-Ala-Arg-Glu-Ala-Ala-Ala-Arg-Asp-Ala-Ala-Ala-Lys (SEQ ID NO:7); and
e) Asn-Val-Asp-His-Lys-Pro-Ser-Asn-Thr-Lys-Val-Asp-Lys-Arg (SEQ ID NO:8).

3. A polynucleotide encoding the heterologous fusion protein of claim 1 or claim 2.

4. A vector comprising the polynucleotide of claim 3.

5. A host cell comprising the vector of claim 4.

6. A host cell expressing the heterologous fusion protein of claim 1 or claim 2.

7. The host cell of claim 6 wherein the host cell is a CHO cell.

8. The host cell of claim 6 wherein the host cell is a NSO cell.

9. A process for producing a heterologous fusion protein comprising the steps of transcribing and translating a polynucleotide of claim 3 under conditions wherein the heterologous fusion protein is expressed in detectable amounts.

10. The heterologous fusion protein of claim 1 or claim 2 for use as a medicament.

## Patentansprüche

1. Heterologes Fusionsprotein, umfassend ein aktives therapeutisches Peptid, das an den Fc Teil eines Immunglobulins fusioniert ist, wobei der Fc Teil die folgende Sequenz SEQ ID NO:1 umfasst worin
Xaa an der Position 1 für Ala steht oder fehlt,
Xaa an der Position 16 für Pro oder Glu steht,
Xaa an der Position 17 für Val oder Ala steht,
Xaa an der Position 18 für Ala steht,
Xaa an der Position 80 für Asn oder Ala steht, und
Xaa an der Position 230 für Lys steht oder fehlt.

2. Heterologes Fusionsprotein nach Anspruch 1, worin die C-terminale Aminosäure des aktiven therapeutischen Peptids an den N-terminalen Rest des Fc Teils über einen Peptidlinker fusioniert ist, der eine Sequenz umfasst, die ausgewählt ist aus:
a) Gly-Gly-Gly-Gly-Ser-Gly-Gly-Gly-Gly-Ser-Gly-Gly-Gly-Gly-Ser (SEQ ID NO:2);
b) Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:4);
c) Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:6);
d) Asp-Ala-Ala-Ala-Lys-Glu-Ala-Ala-Ala-Lys-Asp-Ala-Ala-Ala-Arg-Glu-Ala-Ala-Ala-Arg-Asp-Ala-Ala-Ala-Lys (SEQ ID NO:7); und
e) Asn-Val-Asp-His-Lys-Pro-Ser-Asn-Thr-Lys-Val-Asp-Lys-Arg (SEQ ID NO:8).

3. Polynucleotid, das für das heterologe Fusionsprotein nach Anspruch 1 oder 2 kodiert.

4. Vektor, umfassend das Polynucleotid nach Anspruch 3.

5. Wirtszelle, umfassend den Vektor nach Anspruch 4.

6. Wirtszelle, zur Expression des heterologen Fusionsproteins nach Anspruch 1 oder 2.

7. Wirtszelle nach Anspruch 6, worin diese Zelle eine CHO Zelle ist.

8. Wirtszelle nach Anspruch 6, worin diese Zelle eine NSO Zelle ist.

9. Verfahren zur Herstellung eines heterologen Fusionsproteins, umfassend die Stufen einer Transkription und Translation eines Polynucleotids nach Anspruch 3 unter solchen Bedingungen, dass das heterologe Fusionsprotein in detektierbaren Mengen exprimiert wird.

10. Heterologes Fusionsprotein nach Anspruch 1 oder 2 zur Verwendung als ein Arzneimittel.

## Revendications

1. Protéine fusionnée hétérologue comprenant un peptide thérapeutiquement actif fusionné à la portion Fc d'une immunoglobuline, la portion Fc comprenant la séquence SEQ ID NO :1 où :
Xaa en position 1 représente Ala ou est absent ;
Xaa en position 16 représente Pro ou Glu ;
Xaa en position 17 représente Val ou Ala ;
Xaa en position 18 représente Ala ;
Xaa en position 80 représente Asn ou Ala et
Xaa en position 230 représente Lys ou est absent.

2. Protéine fusionnée hétérologue selon la revendication 1 où l'acide aminé C-terminal du peptide thérapeutiquement actif est fusionné au résidu N-terminal de la portion Fc via un lieur peptidique comprenant une séquence choisie parmi :
a) Gly-Gly-Gly-Ser-Gly-Gly-Gly-Gly-Ser-Gly-Gly-Gly-Gly-Ser (SEQ ID NO:2);
b) Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:4);
c) Gly-Gly-Gly-Gly-Ser-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO:6);
d) Asp-Ala-Ala-Ala-Lys-Glu-Ala-Ala-Ala-Lys-Asp-Ala-Ala-Ala-Arg-Glu-Ala-Ala-Ala-Arg-Asp-Ala-Ala-Ala-Lys (SEQ ID NO:7); et
e) Asn-Val-Asp-His-Lys-Pro-Ser-Asn-Thr-Lys-Val-Asp-Lys-Arg (SEQ ID NO:8).

3. Polynucléotide codant pour la protéine fusionnée hétérologue selon la revendication 1 ou la revendication 2.

4. Vecteur comprenant le polynucléotide selon la revendication 3.

5. Cellule hôte comprenant le vecteur selon la revendication 4.

6. Cellule hôte exprimant la protéine fusionnée hétérologue selon la revendication 1 ou la revendication 2.

7. Cellule hôte selon la revendication 6 où la cellule hôte est une cellule CHO.

8. Cellule hôte selon la revendication 6 où la cellule hôte est une cellule NSO.

9. Procédé pour la production d'une protéine fusionnée hétérologue comprenant les étapes de transcription et de traduction d'un polynucléotide selon la revendication 3 dans des conditions dans lesquelles la protéine fusionnée hétérologue est exprimée en des quantités détectables.

10. Protéine fusionnée hétérologue selon la revendication 1 ou la revendication 2 destinée à une utilisation comme médicament.
